# EUROPEAN PATENT SPECIFICATION

(11) **EP 2 331 066 B1**
(45) Date of publication and mention of the grant of the patent: **09.04.2014**
(21) Application number: 09741420.5
(22) Date of filing: 28.09.2009
(51) Int. Cl.: A61K 9/00, A61K 9/06, A61K 31/728, A61K 47/02, A61K 47/34, A61K 47/36, A61K 47/38, A61L 12/14, A61K 31/785

(54) **MUCOMIMETIC COMPOSITIONS AND USES THEREFORE**
MUCOMIMETISCHE ZUSAMMENSETZUNGEN UND IHRE VERWENDUNG
COMPOSITIONS MUCOMIMETIQUES ET LEUR UTILISATION

(30) Priority: 02.10.2008 US 285350
(43) Date of publication of application: 15.06.2011
(73) Proprietor: CIS Pharma AG, 4416 Bubendorf (CH)
(72) Inventor: SCHAEFER, Rolf, CH-4422 Arisdorf (CH)
(74) Representative: Völlmy, Lukas
(86) International application number: PCT/IB2009/006972
(87) International publication number: WO 2010/038129

(56) References cited:
- WO-A1-03/053453
- US-A1- 2005 245 484
- US-A1- 2006 103 807
- US-A1- 2007 264 226
- MUELLER GERALD ET AL: "In vitro action of a combination of selected antimicrobial agents and chondroitin sulfate" CHEMICO-BIOLOGICAL INTERACTIONS, vol. 124, no. 2, 15 January 2000 (2000-01-15), pages 77-85, XP002558609 ISSN: 0009-2797

## Description

### FIELD OF THE INVENTION

The invention relates to mucomimetic compositions wherein a magnesium, calcium or magnesium/calcium complex of an anionic polymeric substance, especially hyaluronic acid, carboxymethylcellulose, alginate and/or chondroitin sulfate, is combined with a cationic polymeric antimicrobial agent such as polyaminopropyl biguanide, wherein said compositions provide comfort and biocompatibility with mucous membranes without substantially affecting antimicrobial efficacy of the cationic polymeric antimicrobial agent.

### BACKGROUND OF THE INVENTION

Anionic polymers such as hyaluronic acid and carboxymethylcellulose are known for their moisturizing and lubricating properties. Such properties generally result in a reduction in irritation to mucous membranes, including those of the eye. Cationic antimicrobial agents have also been noted as beneficial to include in ophthalmic solutions. However, these two types of components were not understood as being compatible within one solution.

Cationic antimicrobial disinfecting agents were found to be compromised in their efficacy in the presence of certain anionic entities. For example, U.S. Pat. No. 5,858,346 teaches that polyaminopropyl biguanide and other non-oxidative disinfectants (typically cationic entities) are neutralized in their ability to damage cell walls, including cell walls of microorganisms, when combined with carboxymethyl cellulose and/or various other negatively charged entities. Although the neutralization of the polyaminopropyl biguanide or other non-oxidative disinfectants alleviates irritation of mucous membranes (i.e., the eye), the neutralization also results in loss of antimicrobial efficacy.

Similarly, U.S. Pat. No. 5,559,104, to Romeo et al., teaches the use of cetylpyridinium chloride as an ion-pairing agent for the precipitation and purification of hyaluronic acid in a proposed manufacturing process for the hyaluronic acid biopolymer. Thus, that cetylpyridinium chloride would retain its antimicrobial activity in the presence of hyaluronic acid or carboxymethyl cellulose is counterintuitive. However, Powell and Huth were recently able to prepare ophthalmic solutions comprising an anionic polymer and a cationic monomeric or dimeric antimicrobial agent, in which activity of the antimicrobial agent was retained. U.S. Pat. Appl. No. 11/271,448. It is noted that cationic polymeric antimicrobial agents were discussed. However, no composition containing such antimicrobial agent was proposed, suggesting that the authors were unable to or considered it not possible to prepare such a composition without concomitant loss of antimicrobial activity. Mackles disclosed solutions comprising an anionic polymer, a quaternary germicide (i.e., benzalkonium chloride) and polysorbate (U.S. patent publication no. 2005/245484 A1). Neutralization of the cationic quaternary germicide was overcome by the addition of the surfactant. Lloyd disclosed eye-instillable formulations comprising polyaminopropyl biguanide and sodium hyaluronate in which formulations the quaternary polymeric agent was claimed to have retained antimicrobial activity (International patent publication no. WO 03/053453). No explanation was provided for why the cationic antimicrobial agent was not neutralized by the anionic polymer. That such neutralization actually occurs had been reported previously (Müller and Kramer (2000) Chemico-Biological Interactions 124: 77-85).

### SUMMARY OF THE INVENTION

The present invention relates to mucomimetic solutions comprising a cationic polymeric antimicrobial agent in an amount ranging from about 0.001 to about 0.01% w/v and a magnesium, calcium or magnesium/calcium complex of an anionic polymer in an amount ranging from about 0.01 to about 0.25% w/v, wherein the cationic polymeric antimicrobial agent is polyaminopropyl biguanide or an acceptable salt thereof. The anionic polymer may be selected from the group consisting of hyaluronate, alginate, carboxymethyl cellulose, chondroitin sulfate and mixtures thereof. In more preferred embodiments, the anionic polymer has a molecular weight of from about 70,000 to about 4 million Daltons. The mucomimetic solutions of the invention may include one or more additional components such as a viscosity-modifying agent, preferably selected from the group consisting of hydroxypropylmethyl cellulose, carboxymethyl cellulose and hydroxyethyl cellulose, in an amount ranging from about 0.01 to about 0.2%, a surfactant in an amount ranging from about 0.01 to about 1% w/v, a buffer in an amount ranging from about 0.01 to about 0.25% w/v or a tonicity agent in an amount ranging from about 0.001 to about 1% w/v. A preferred surfactant is polysorbate 20, which is included in an amount ranging from about 0.01 to about 1% w/v.

Other embodiments of the invention relate to ophthalmic solutions comprising a cationic polymeric antimicrobial agent in an amount ranging from about 0.0001 to about 0.0005% w/v and a magnesium, calcium or magnesium/calcium complex of an anionic polymer in an amount ranging from about 0.01 to about 0.25% w/v, wherein the cationic polymeric antimicrobial agent is polyaminopropyl biguanide or an acceptable salt thereof. The anionic polymer may be selected from the group consisting of hyaluronate, alginate, carboxymethyl cellulose, chondroitin sulfate and mixtures thereof. In more preferred embodiments, the anionic polymer has a molecular weight of from about 70,000 to about 4 million Daltons. The ophthalmic solutions of the invention may include one or more additional components such as a viscosity-modifying agent, preferably selected from the group consisting of hydroxypropylmethyl cellulose, carboxymethyl cellulose and hydroxyethyl cellulose, in an amount ranging from about 0.01 to about 0.2%, a surfactant in an amount ranging from about 0.01 to about 1% w/v, a buffer in an amount ranging from about 0.01 to about 0.25% w/v or a tonicity agent in an amount ranging from about 0.001 to about 1% w/v. A preferred surfactant is polysorbate 20, which is included in an amount ranging from about 0.01 to about 1% w/v.

A more specific embodiment of the ophthalmic solution of the present invention is a solution comprising polyaminopropyl biguanide in an amount ranging from about 0.0001 to about 0.0005% w/v, a magnesium, calcium or magnesium/calcium complex of an anionic polymer in an amount ranging from about 0.01 to about 0.25% w/v, a viscosity-modifying agent in an amount ranging from about 0.01 to about 0.2% w/v, polysorbate 20 in an amount ranging from about 0.01 to about 1% w/v and a tonicity agent in an amount ranging from about 0.001 to about 1% w/v. The ophthalmic solution may further include a buffer in an amount ranging from about 0.01 to about 0.25% w/v. In a more preferred embodiment, the anionic polymer has a molecular weight of from about 70,000 to about 4 million Daltons. The most preferred anionic polymer is hyaluronate. In more specific embodiments, the viscosity-modifying agent is hydroxypropylmethyl cellulose and the tonicity agent is sodium chloride or a mixture of sodium chloride and potassium chloride.

Provided also are mucomimetic solution of the invention, for use in wipes, solutions or lubricating products that are brought in contact with mucous membranes of a subject. Provided also are ophthalmic solutions of the invention for use in contact lens disinfection compositions, lens cleansing solutions compositions, lens packaging solution compositions or eye drops.

### DETAILED DESCRIPTION OF THE INVENTION

Novel mucomimetic solutions comprising a complex of a negatively charged polymeric substance and a cationic polymeric antimicrobial agent are described, as well as their methods of use and preparation. Solutions according to the present invention may be used as ophthalmic solutions for effective contact lens disinfection compositions, lens cleansing solution compositions, lens packaging solution compositions, eye drops such as rewetters and tears or as vehicles for topical delivery of active substances to the eye. The compositions provide additional comfort to the eye. Furthermore, they are biocompatible with lenses and, unlike prior art compositions, do not cause a discoloring of soft contact lenses. The efficacy of the antimicrobial agent is retained in the compositions of the invention. The compositions of the invention may also be used in wipes, solutions and lubricating products that are brought in contact with other mucous membranes of a subject. They can be used for moisturing, lubricating, cleaning or disinfection purposes and may also serve as vehicles for delivering an active substance into or through the latter mucous membranes.

It has been discovered that certain anionic polymeric substances which form supramolecular complexes in the presence of magnesium or calcium ions, are highly comfortable to the eye and do not inactivate cationic polymeric antimicrobial agents such as polyaminopropyl biguanide, provided that the concentration (w/v) of the cationic polymeric antimicrobial agent is at least about ten fold lower than that of the anionic polymer. The anionic polymeric substances used in the solutions of the invention include hyaluronate, alginate, carboxymethyl cellulose, chondroitin sulfate and mixtures thereof.

The compositions of the invention are compatible with typical additives to mucomimetic and ophthalmic compositions, such as typical buffer systems, surfactants, tonicity agents and viscosity-modifying agents.

In one embodiment, a composition of the invention comprises a complex of at least one anionic polymeric substance, such as hyaluronate, alginate, carboxymethyl cellulose, chondroitin sulfate or mixtures thereof, and at least one cationic polymeric antimicrobial agent such as polyaminopropyl biguanide or an acceptable salt thereof.

In a preferred embodiment, the composition is comprised of specific concentration ranges of said cationic polymeric antimicrobial agent, preferably polyaminopropyl biguanide or an acceptable salt thereof, and said complex of a water-soluble anionic polymeric substance. A typical concentration range for the cationic polymeric antimicrobial agent is from about 0.001% to about 0.01% w/v for a general purpose solution and from about 0.0001 to about 0.0005% w/v for an ophthalmic solution. The range for the complexed anionic polymeric substance is from about 0.01% to about 0.25% w/v. Preferably, the anionic polymeric substance has a molecular weight of from about 70'000 to about 4 million Daltons.

In a more preferred embodiment, a mucomimetic or ophthalmic composition of the invention is comprised of polyaminopropyl biguanide or an acceptable salt thereof such as a borate salt, and a magnesium, calcium or magnesium/calcium (any ratio) complex of hyaluronate.

The compositions of the present invention typically include a buffer component. The present compositions may have a pH that is compatible with the intended use, and is often between about 5 and 9. A variety of conventional buffers may be employed, such as borate, citrate, acetate, histidine, tris, bis-tris and the like and mixtures thereof. Borate buffers include boric acid and its salts, such as sodium or potassium borate. Potassium tetraborate or potassium metaborate, which produce boric acid or a salt of boric acid in solution, may also be employed. Hydrated salts such as sodium borate decahydrate can also be used. Additionally, organic counter-ions for the above buffers may also be employed. The concentration of buffer generally ranges from about 0.01 to 0.25% w/v.

The type and amount of buffer are selected so that the composition meets the functional performance criteria of the composition, such as physicochemical attributes and shelf life stability, antimicrobial efficacy, buffer capacity and the like factors. The buffer is also selected to provide a pH, which is compatible with the target mucous membranes such as those of the eye and any contact lenses with which the composition is intended to be used. Generally, for ophthalmic compositions a pH close to that of human tears, such as a pH of about 7.45, is very useful, although a wider pH range from about 5 to about 9, more preferably about 6.5 to about 8.5 and still more preferably about 7.0 to about 8.0 is also acceptable. These pH values and ranges generally also apply to mucomimetic compositions that are intended for administration to other mucous membranes.

The osmolarity of the present mucomimetic compositions may be adjusted with tonicity agents to a value that is compatible with the intended use of the compositions. For example, the osmolarity of ophthalmic solutions of the invention may be adjusted to approximately the osmotic pressure of normal tear fluid, which is equivalent to about 0.9 w/v % of sodium chloride in water. Examples of suitable tonicity adjusting agents include, without limitation: chloride salts of sodium, potassium, calcium and magnesium; dextrose; glycerol; propylene glycol; mannitol; sorbitol and the like; and mixtures thereof. Preferred tonicity agents are sodium chloride or combinations of sodium chloride and potassium chloride.

Tonicity agents are typically used in amounts ranging from about 0.001 to about 1 % w/v. These amounts have been found to be useful in providing a physiologically acceptable tonicity. Preferably, the tonicity agent(s) will be employed in an amount to provide a final osmotic value of 150 to 450 mOsm/kg, more preferably between about 220 to about 350 mOsm/kg and most preferably between about 270 to about 310 mOsm/kg.

The compositions of the invention, particularly those that are intended for use as lens disinfection compositions, lens cleansing solutions, or lens packaging solution compositions, may be further supplemented with one or more surfactants. Such surfactants may include polyethylene oxide (PEO), polypropylene oxide (PPO), or block copolymers comprised of combinations of these. A preferred surfactant for inclusion with compositions of the invention is polysorbate 20. Inclusion of such surfactants results in effective lens cleaning during lens treatment without substantially affecting the antimicrobial activity of the compositions. The concentration of surfactants in the compositions typically ranges from about 0.01 to about 1% w/v.

The compositions of the invention may further include one or more viscosity-modifying agents such as cellulose polymers, including hydroxypropylmethyl cellulose, hydroxyethyl cellulose, ethylhydroxyethyl cellulose, hydroxypropyl cellulose, methyl cellulose, carboxymethyl cellulose, glycerol, carbomers, polyvinyl alcohol, polyvinyl pyrrolidone, alginates, carrageenans, guar, karaya, agarose, locust bean, tragacanth and xanthan gums. Such viscosity modifying components are typically employed in an amount effective to provide a desired lubricating effect to the present compositions. The concentration of such viscosity-modifying agents will typically be between about 0.01 and 0.2% w/v. Preferred viscosity modifying agents are hydroxypropylmethyl cellulose, carboxymethyl cellulose and hydroxyethyl cellulose.

A preferred composition of the present invention to be used as an ophthalmic solution comprises polyaminopropyl biguanide in an amount ranging from about 0.0001 to about 0.0005% w/v, a magnesium, calcium or magnesium/calcium complex of an anionic polymer in an amount ranging from about 0.01 to about 0.25% w/v, a viscosity-modifying agent in an amount ranging from about 0.01 to about 0.2% w/v, polysorbate 20 in an amount ranging from about 0.01 to about 1% w/v and a tonicity agent in an amount ranging from about 0.001 to about 1% w/v. The most preferred ophthalmic solution also comprises a buffer, preferably a borate buffer. In this most preferred ophthalmic solution, the anionic polymer is hyaluronate, preferably of a molecular weight from about 70'000 to about 4 million Dalton, the viscosity-modifying agent is hydroxypropylmethyl cellulose and the toniciy agent is sodium chloride or a combination of sodium chloride and potassium chloride.

Methods for using the ophthalmic solutions described herein are considered to be within the scope of the present invention. The mucomimetic compositions of the invention may be used in wipes, solutions, lubricating products and the like that are brought in contact with other mucous membranes of a subject. They may serve to moisturize, lubricate, clean and/or disinfect. They may also be used as non-irritating, preservative or disinfecting carriers of drug substances for delivery of the drug substances into or through mucous membranes. When used as ophthalmic solutions, the compositions may also be utilized as contact lens disinfection compositions, lens cleansing solution compositions, lens packaging solution compositions or eye drops such as rewetters and tears. The compositions are highly comfortable to the eye and are biocompatible with contact lenses. The polymeric anionic and cationic compounds are known not to penetrate into the lens matrix. Unlike prior art compositions, they do not cause a discoloring of soft contact lenses, especially of silicon hydrogel lenses.

The invention is further elaborated by the following examples. The examples are provided for purposes of illustration to a person skilled in the art and are not intended to be limiting the scope of the invention as described in the claims. Thus, the invention should not be construed as being limited to the examples provided, but should be construed to encompass any and all variations that become evident as a result of the teaching provided herein.

### EXAMPLES

### Components of the compositions of the invention

All components of the mucomimetic compositions of the present invention can be obtained from commercial sources. Polyaminopropyl biguanide can be procured from Thor Specialties, Inc. (Trumbull, Connecticut, USA). All other components are available from, e.g., from Sigma-Aldrich Fluka (Buchs, Switzerland).

### Example 1: Antimicrobial activity of polyaminopropyl biguanide in the presence of calcium/magnesium salts of anionic polymers.

Formulations: 0.1 % hyaluronic acid (A) or 0.15% carboxymethylcellulose (B), or 0.05% alginate (C); 0.01% polyaminopropyl biguanide; 0.02% calcium chloride; 0.02% magnesium chloride; 0.1 % sodium hydrogenborate; pH 7.2.

Parallel cultures of different microorganisms were either exposed to formulations A, B or C for 24 hours or were incubated further in their respective growth media. At the end of the challenge period, bacterial counts (per ml) were determined.

| Organism | Challenge formulation A | challenge formulation B | Challenge formulation C | No exposure | No exposure | No exposure |
|---|---|---|---|---|---|---|
| B.subtitlis | <10 | <10 | <10 | 5.8 x 10⁶ | 5.8 x 10⁶ | 5.8 x 10⁶ |
| P.aeruginosa | <10 | <10 | <10 | 3.7 x 10⁶ | 3.7 x 10⁶ | 3.7 x 10⁶ |
| S.aureus | <10 | <10 | <10 | 5.4 x 10⁶ | 5.4 x 10⁶ | 5.4 x 10⁶ |
| C.albicans | <10² | <10² | <10² | 1.3 x 10⁶ | 1.3 x 10⁶ | 1.3 x 10⁶ |

### Example 2: Formulation of a general mucomimetic lubricant/wettening ointment.

An aqueous solution is prepared by mixing 0.01% hyaluronic acid sodium, 0.005% polyaminopropyl biguanide , 0.025% calcium chloride, 0.2% HPMC, 10% glycerol; 0.5% sodium hydrogenborate. The solution is adjusted to pH 7.2.

### Example 3: Formulation of a multipurpose contact lens solution.

An aqueous solution is prepared by mixing 0.1% hyaluronic acid sodium, 0.00025% polyaminopropyl biguanide, 0.02% calcium chloride, 0.02% magnesium chloride, 0.1% HPMC, 0.025% polysorbate 20, 0.3% sodium chloride, 0.2% sodium hydrogenborate. The solution is adjusted to pH 7.2.

### Example 4: Formulation of a dry eye ointment.

An aqueous solution is prepared by mixing 0.2% hyaluronic acid sodium, 0.0003% polyaminopropyl biguanide, 0.025% calcium chloride, 0.15% HPMC, 0.2% sodium chloride, 0.3% sodium hydrogenborate. The solution is adjusted to pH 7.0.

### Example 5: Formulation of a glaucoma ointment.

An aqueous solution is prepared by mixing 0.25% timolol maleate ((-)-1-(*tert-*butylamino)-3-[(4-morpholino-1,2,5-thiadiazol-3-yl)oxy]-2-propanol maleate (1:1) (salt)), 0.25% hyaluronic acid sodium, 0.0002% polyaminopropyl biguanide - borate, 0.4% sodium chloride, 0.026% calcium chloride, 0.1% sodium hydrogenborate. The solution is adjusted to pH 6.5.

## Claims

1. An ophthalmic solution comprising a cationic polymeric antimicrobial agent in an amount ranging from about 0.0001 to about 0.0005% w/v and a magnesium, calcium or magnesium/calcium complex of an anionic polymer in an amount ranging from about 0.01 to about 0.25% w/v, wherein the cationic polymeric antimicrobial agent is polyaminopropyl biguanide or an acceptable salt thereof.

2. The ophthalmic solution of claim 1 wherein the anionic polymer has a molecular weight of from about 70,000 to about 4 million Daltons.

3. The ophthalmic solution of claims 1-2 wherein the anionic polymer is selected from the group consisting of hyaluronate, alginate, carboxymethyl cellulose, chondroitin sulfate and mixtures thereof.

4. The ophthalmic solution of claims 1-3 further including a viscosity-modifying agent selected from the group consisting of hydroxypropylmethyl cellulose, carboxymethyl cellulose and hydroxyethyl cellulose in an amount ranging from about 0.01 to about 0.2% w/v.

5. The ophthalmic solution of claims 1-4 further including a surfactant in an amount ranging from about 0.01 to about 1% w/v.

6. The ophthalmic solution of claims 1-5 further including polysorbate 20 in an amount ranging from about 0.01 to about 1% w/v.

7. The ophthalmic solution of claims 1-6 further including a buffer in an amount ranging from about 0.01 to about 0.25% w/v.

8. The ophthalmic solution of claims 1-7 further including a tonicity agent in an amount ranging from about 0.001 to about 1% w/v.

9. The ophthalmic solution of claim 1, further comprising a viscosity-modifying agent in an amount ranging from about 0.01 to about 0.2% w/v, polysorbate 20 in an amount ranging from about 0.01 to about 1% w/v and a tonicity agent in an amount ranging from about 0.001 to about 1% w/v.

10. The ophthalmic solution of claim 9 further including a buffer in an amount ranging from about 0.01 to about 0.25% w/v.

11. The ophthalmic solution of claims 9-10 wherein the anionic polymer has a molecular weight of from about 70,000 to about 4 million Daltons.

12. The ophthalmic solution of claims 9-11 wherein the anionic polymer is hyaluronate.

13. The ophthalmic solution of claims 9-12 wherein the viscosity-modifying agent is hydroxypropylmethyl cellulose and the tonicity agent is sodium chloride or a mixture of sodium chloride and potassium chloride.

14. The ophthalmic solution of any one of the above claims, for use in contact lens disinfection compositions, lens cleansing solutions compositions, lens packaging solution compositions or eye drops.

15. A mucomimetic solution comprising a cationic polymeric antimicrobial agent in an amount ranging from about 0.001 to about 0.01 % w/v and a magnesium, calcium or magnesium/calcium complex of an anionic polymer in an amount ranging from about 0.01 to about 0.25% w/v, wherein the cationic polymeric antimicrobial agent is polyaminopropyl biguanide or an acceptable salt thereof, and wherein the concentration (w/v) of the cationic polymeric antimicrobial agent is at least about ten fold lower than that of the anionic polymer.

16. The mucomimetic solution of claim 15, for use in wipes, solutions or lubricating products that are brought in contact with mucous membranes of a subject.

## Patentansprüche

1. Eine ophthalmische Lösung welche ein kationisches, antimikrobielles Polymer in einer Menge von ungefähr 0.0001 bis ungefähr 0.0005% (Gewicht/Volumen) und ein mit Magnesium, Kalzium oder Magnesium/Kalzium komplexiertes anionisches Polymer in einer Menge von ungefähr 0.01 bis ungefähr 0.25% (Gewicht/Volumen) enthält, wobei es sich beim kationischen Polymer um Polyaminopropylbiguanid oder ein akzeptables Salz davon handelt.

2. Die ophthalmische Lösung von Anspruch 1, wobei das anionische Polymer ein Molekulargewicht von ungefähr 70'000 bis ungefähr 4 Millionen Dalton hat.

3. Die ophthalmische Lösung von Anspruch 1 oder 2, wobei das anionische Polymer aus einer Gruppe ausgewählt ist, bestehend aus Hyaluronat, Alginat, Carboxymethylcellulose, Chondroitinsulfat und deren Mischungen.

4. Die ophthalmische Lösung nach einem der Ansprüche 1 bis 3, **dadurch gekennzeichnet, dass** die Lösung zusätzlich eine Menge von ungefähr 0.01 bis ungefähr 0.2% (Gewicht/Volumen) eines Viskositätsmodifizierungsmittels einschliesst, welches ausgewählt ist aus der Gruppe bestehend aus Hydroxypropylmethylcellulose, Carboxymethylcellulose und Hydroxyethylcellulose.

5. Die ophthalmische Lösung nach einem der Ansprüche 1 bis 4, **dadurch gekennzeichnet, dass** die Lösung zusätzlich eine Menge von ungefähr 0.01 bis ungefähr 1 % (Gewicht/Volumen) eines Benetzungsmittels einschliesst

6. Die ophthalmische Lösung nach einem der Ansprüche 1 bis 5, **dadurch gekennzeichnet, dass** die Lösung zusätzlich eine Menge von ungefähr 0.01 bis ungefähr 1 % (Gewicht/Volumen) Polysorbat 20 einschliesst.

7. Die ophthalmische Lösung nach einem der Ansprüche 1 bis 6, **dadurch gekennzeichnet, dass** die Lösung zusätzlich eine Menge von ungefähr 0.01 bis ungefähr 0.25% (Gewicht/Volumen) eines Puffers einschliesst.

8. Die ophthalmische Lösung nach einem der Ansprüche 1 bis 7, **dadurch gekennzeichnet, dass** die Lösung zusätzlich eine Menge von ungefähr 0.001 bis ungefähr 1 % (Gewicht/Volumen) eines Tonizitätsmittels einschliesst.

9. Die ophthalmische Lösung von Anspruch 1, **dadurch gekennzeichnet, dass** die Lösung zusätzlich ein Viskositätsmodifizierungsmittel in einer Menge von ungefähr 0.01 bis ungefähr 0.2% (Gewicht/Volumen), Polysorbat 20 in einer Menge von ungefähr 0.01 bis ungefähr 1% (Gewicht/Volumen) und ein Tonizitätsmittel in einer Menge von ungefähr 0.001 bis ungefähr 1 % (Gewicht/Volumen) einschliesst.

10. Die ophthalmische Lösung von Anspruch 9, **dadurch gekennzeichnet, dass** die Lösung zusätzlich eine Menge von ungefähr 0.01 bis ungefähr 0.25% (Gewicht/Volumen) eines Puffers einschliesst.

11. Die ophthalmische Lösung von Anspruch 9 oder 10, wobei das anionische Polymer ein Molekulargewicht von ungefähr 70'000 bis ungefähr 4 Millionen Dalton hat.

12. Die ophthalmische Lösung nach einem der Ansprüche 9 bis 11, wobei das anionische Polymer Hyaluronat ist.

13. Die ophthalmische Lösung nach einem der Ansprüche 9 bis 12, wobei das Viskositätsmodifizierungsmittel Hydroxypropylmethylcellulose ist und das Tonizitätsmittel Natriumchlorid oder eine Mischung von Natriumchlorid und Kaliumchlorid.

14. Die ophthalmische Lösung nach irgendeinem der obengenannten Ansprüche, zur Benützung als Linsendesinfektionsmittel, Linsenreinigungsmittel, Linsenverpackungslösung oder Augentropfen.

15. Eine mukomimetische Lösung welche ein kationisches, antimikrobielles Polymer in einer Menge von ungefähr 0.001 bis ungefähr 0.01% (Gewicht/Volumen) und ein mit Magnesium, Kalzium oder Magnesium/Kalzium komplexiertes anionisches Polymer in einer Menge von ungefähr 0.01 bis ungefähr 0.25% (Gewicht/Volumen) enthält, wobei es sich beim kationischen Polymer um Polyaminopropylbiguanid oder ein akzeptables Salz davon handelt, und wobei die Konzentration (Gewicht/Volumen) des kationischen, antimikrobiellen Polymers mindestens ungefähr zehnfach tiefer ist als diejenige des anionischen Polymers.

16. Die mucomimetische Lösung von Anspruch 15, zur Benützung in Wischtüchern, Lösungen oder Gleitmitteln welche mit mukösen Membranen einer Person in Berührung kommen.

## Revendications

1. Solution ophtalmique comprenant un agent antimicrobien polymère cationique selon une quantité allant d'environ 0,0001 à environ 0,0005% p/v et un complexe de magnésium, calcium ou magnésium/calcium d'un polymère anionique selon une quantité allant d'environ 0,01 à environ 0,25% p/v, dans laquelle l'agent antimicrobien polymère cationique est un biguanide de polyaminopropyle ou un sel pharmaceutiquement acceptable de celui-ci.

2. Solution ophtalmique selon la revendication 1, dans laquelle le polymère anionique possède un poids moléculaire allant d'environ 70 000 à environ 4 millions de Daltons.

3. Solution ophtalmique selon les revendications 1-2, dans laquelle le polymère anionique est choisi dans le groupe constitué par un hyaluronate, un alginate, la carboxyméthylcellulose, le sulfate de chondroïtine et des mélanges de ceux-ci.

4. Solution ophtalmique selon les revendications 1-3, comportant en outre un agent de modification de la viscosité choisi dans le groupe constitué par l'hydroxypropyl méthylcellulose, la carboxyméthylcellulose et l'hydroxyéthylcellulose, selon une quantité allant d'environ 0,01 à environ 0,2% p/v.

5. Solution ophtalmique selon les revendications 1-4, comportant en outre un agent tensioactif selon une quantité allant d'environ 0,01 à environ 1% p/v.

6. Solution ophtalmique selon les revendications 1-5, comportant en outre du polysorbate 20 selon une quantité allant d'environ 0,01 à environ 1% p/v.

7. Solution ophtalmique selon les revendications 1-6, comportant en outre un tampon selon une quantité allant d'environ 0,01 à environ 0,25% p/v.

8. Solution ophtalmique selon les revendications 1-7, comportant en outre un agent de tonicité selon une quantité allant d'environ 0,001 à environ 1% p/v.

9. Solution ophtalmique selon la revendication 1, comportant en outre un agent de modification de la viscosité selon une quantité allant d'environ 0,01 à environ 0,2% p/v, du polysorbate 20 selon une quantité allant d'environ 0,01 à environ 1% p/v, et un agent de tonicité selon une quantité allant d'environ 0,001 à environ 1% p/v.

10. Solution ophtalmique selon la revendication 9, comportant en outre un tampon selon une quantité allant d'environ 0,01 à environ 0,25% p/v.

11. Solution ophtalmique selon les revendications 9-10, dans laquelle le polymère anionique possède un poids moléculaire allant d'environ 70 000 à environ 4 millions de Daltons.

12. Solution ophtalmique selon les revendications 9-11, dans laquelle le polymère anionique est un hyaluronate.

13. Solution ophtalmique selon les revendications 9-12, dans laquelle l'agent de modification de la viscosité est l'hydroxypropyl méthylcellulose, et l'agent de tonicité est le chlorure de sodium ou un mélange de chlorure de sodium et de chlorure de potassium.

14. Solution ophtalmique selon l'une quelconque des revendications précédentes, pour une utilisation dans des compositions de désinfection de lentilles de contact, des compositions de solutions de nettoyage de lentilles, des compositions de solutions d'emballage de lentilles ou des gouttes ophtalmiques.

15. Solution mucomimétique, comprenant un agent antimicrobien polymère cationique selon une quantité allant d'environ 0,001 à environ 0,01% p/v et un complexe de magnésium, calcium ou magnésium/calcium d'un polymère anionique selon une quantité allant d'environ 0,01 à environ 0,25% p/v, dans laquelle l'agent antimicrobien polymère cationique est un biguanide de polyaminopropyle ou un sel pharmaceutiquement acceptable de celui-ci, et dans laquelle la concentration (p/v) en agent antimicrobien polymère cationique est au moins environ dix fois inférieure à celle du polymère anionique.

16. Solution mucomimétique selon la revendication 15, pour une utilisation dans des lingettes, des solutions ou des produits lubrifiants qui sont mis en contact avec les membranes muqueuses d'un sujet.
